# EUROPEAN PATENT SPECIFICATION

(11) **EP 1 218 027 B1**
(45) Date of publication and mention of the grant of the patent: **20.01.2010**
(21) Application number: 00965007.8
(22) Date of filing: 15.09.2000
(51) Int. Cl.: A61K 38/39, A61K 38/17, A61K 38/45, A61P 27/02

(54) **COMPOSITION FOR STABILIZING CORNEAL TISSUE DURING OR AFTER ORTHOKERATOLOGICAL LENS WEAR**
ZUSAMMENSETZUNG ZUR STABILISIERUNG DES CORNEAGEWEBES WÄHREND ODER NACH ORTHOKERATOLOGISCHEM TRAGEN VON KONTAKTLINSEN
COMPOSITION DE STABILISATION DU TISSU DE LA CORNEE PENDANT OU APRES LE PORT DE LENTILLE D'ORTHOKERATOLOGIE

(30) Priority: 15.09.1999 US 153959 P; 30.12.1999 US 173801 P
(43) Date of publication of application: 03.07.2002
(73) Proprietor: The Bruce H. DeWoolfson Irrevocable Family Trust, West Chester, PA 19380 (US); Euclid Systems Corporation, Herndon, VA 20171 (US)
(72) Inventor: Bruce H, DeWoolfson, Herndon, VA 21071 (US); Dale P. Devore, Chelmsford, MA 01824 (US)
(74) Representative: Viering, Jentschura & Partner
(86) International application number: PCT/US2000/025190
(87) International publication number: WO 2001/019386

(56) References cited:
- US-A- 5 270 051
- US-A- 5 626 865
- DEVORE D P ET AL: "Rapidly polymerized collagen gel as a smoothing agent in excimer laser photoablation." JOURNAL OF REFRACTIVE SURGERY, (1995 JAN-FEB) 11 (1) 50-5. , XP000990182

## Description

### BACKGROUND OF THE INVENTION

### The Field of the Invention

This invention relates to chemical compositions suitable for maintaining corneal curvature resulting from an orthokeratology procedure, and methods for making the chemical compositions to a cornea for retaining the corneal curvature after undergoing orthokeratological therapy and the use thereof for the manufacture of a medicament.

### Description of the Related Art

**Structure and composition of a human cornea**: The cornea is the first and most powerful refracting surface of the optical system of the eye. Production of a sharp image at the retinal receptors requires that the cornea be transparent and of appropriate refractive power. The refractive power of the cornea depends primarily on two factors: its curvature and its refractive index. When the cornea is misshapened or the axial length of the eye is too long or short, or the lens of the eye is functioning abnormally, various vision related problems, such as myopia, astigmatism, hyperopia, or the like, can result. Eyeglasses or contact lenses are necessary to correct the problems. Eyeglasses correct the refractive errors by refracting the light with a lens before it reaches the cornea and to change the angle at which light enters the cornea. Contact lenses correct refractive errors of the eye by replacing the misshapened cornea with a front curve of a contact lens which is calculated to render the eye emmetropic. When the lens is taken off, however, the cornea is still misshapened or defective and refractive errors still remain.

The cornea contains 75% to 80% water on a wet weight basis. Of the remaining 20% to 25% solids, most are collagen, or other proteins, and glycosaminoglycans. Corneal fibrils, which form the skeleton of the corneal stroma, are neatly organized and present the typical 64 to 66 nm periodicity of collagen. The physicochemical properties of corneal collagen, however, do not significantly differ from those of tendon and skin collagen. Like collagen from these other sources, corneal collagen has high nitrogen, glycine, proline, and hydroxyproline contents. In boiling water or acid, corneal collagen is converted to gelatin, and collagen can be dissolved by proteolytic enzymes such as collagenase, pepsin, or papain.

**Orthokeratology procedure**: Orthokeratology is a nonsurgical procedure to improve refractive errors of the eye, and is an alternative to, e.g., laser eye surgery. Specifically, orthokeratology is a therapeutic procedure to reshape the curvature of a patient's cornea. A conventional orthokeratology procedure involves the use of a series of progressive contact lenses that are intended to gradually reshape the cornea and produce a more spherical anterior curvature. The process may involve the fitting of three to six pairs of contact lenses, and it has traditionally taken approximately three to six months to achieve optical reshaping. This procedure has been proven to reduce or eliminate myopia and astigmatism, hence improving natural vision and producing emmetropia (a state where vision experiences zero refractive error, or where no correction is necessary). Recent improvement in orthokeratology lens designs make it possible to achieve emmetropia much more rapidly. In many cases, this may be accomplished with a single night's wear of a single pair of end result lenses.

A problem with orthokeratology is that reshaped corneal tissue keeps a memory of its original curvature, and tends to relax and return to the original curvature after the lenses are removed. Therefore, when an orthokeratology patient reaches maximum results, retainer contact lenses are prescribed for full-time or part-time wear to stabilize the results. The retainer contact lenses have typically been made of rigid gas permeable material. Orthokeratology patients increasingly wear retainer contact lenses during the night to obtain the desired results quickly, and enjoy almost emmetropic vision during their daytime activities. A disadvantage of such a modality is that it requires the wearing of retainer lenses every night in order to keep the cornea from regressing to its former shape.

**Corneoplasty**: A related procedure directed to solve this problem uses a corneal softening agent to temporarily soften the cornea so that it can be more easily reshaped to a desired configuration to produce emmetropia. The corneoplasty procedure is a three-step process performed in one visit or over a period of several weeks. The three-step process includes: first, applying the softening agent to the cornea to soften corneal tissue; second, placing a rigid contact lens over the cornea to render the eye emmetropic; and third, applying a stabilizing agent. The cornea would then reshape and conform to the desired configuration dictated by the rigid contact lens. Administration of the corneal softening agent helps correct larger refractive errors in a shorter period of time.

However, it has been found that it is difficult to accurately place the shaping contact lens with respect to the axis of vision to control the reshaping of the corneal tissue. In some unsuccessful applications, corneoplasty has induced astigmatism or double vision due to errors caused by misplacing the shaping contact lens. In addition, because all three steps are performed in one visit, the patient lacks an opportunity to react to the result of reshaped corneal tissue. The patient cannot "try and see" or change his/her mind during the process.

In light of the foregoing, there is a need for an improved method for performing an acuity correcting procedure that enables the patient to quickly reach emmetropia, while retaining the option of reverting back to his prior level of vision, e.g, to make the procedure be reversible up until the patient chooses to have the correction made permanent. Furthermore, there is a need for compositions and methods for making and applying the composition that stabilizes the cornea matrix. The compositions need to be able to stabilize the corneal curvature resulting from the orthokeratology procedure so that an orthokeratology patient can dispense with wearing rigid retainer contact lenses, dispense with applying a softening agent, and yet retain the opportunity to regress to the original corneal curvature up until the patient is convinced that they want the correction made permanent.

### SUMMARY OF THE INVENTION

The advantages and purposes of the invention will be set forth in part in the description which follows, and in part will be obvious from the description, or may be learned by practice of the invention. The advantages and purposes of the invention will be realized and attained by the elements and combinations particularly pointed out in the appended claims.

To attain the advantages and in accordance with the purposes of the invention, as embodied and broadly described herein, a first aspect of the present invention is directed to the use of a stabilizing agent for the manufacture of a medicament for a therapeutic application to stabilize a reshaped unsoftened corneal tissue which is corrected in an orthokeratological procedure to correct a patient's corneal curvature. The stabilizing agent comprises at least one compound chosen from small leucine-rich repeat proteoglycans ("SLRP"), and fibril associated collagens with interrupted triple-helixes ("FACIT").

A second aspect of the present invention is directed to a method for making a chemical composition for the application on a reshaped cornea resulting from an orthokeratology procedure. The method comprises the steps of preparing transglutaminase in a buffer solution, mixing the solution and diluting in sterile water, adding EDTA to the solution, and adding calcium chloride prior to application of the composition to the cornea.

A third aspect of the present invention is directed to an orthokeratological system, comprising an orthokeratological lens configured to be inserted into a patient's eye to reshape unsoftened corneal tissue into a preselected corneal shape dictated by the orthokeratological lens, and a stabilizing agent administered into the patient's eye to stabilize the corneal tissue in the preselected shape wherein the stabilizing agent comprises at least one compound chosen from transglutaminase, SLRPs, and FACITs.

A fourth aspect of the present invention is directed to a composition suitable for corneal application comprising at least one compound chosen from fibril associated collagens with interrupted triple helices (FACITs) in a physiologically compatible solution. The solution is buffered to a pH of from 6.5 to 8.5, and further wherein the concentration of the FACIT ranges from 10 to 500 µ/ml.

A fifth aspect of the present invention is directed to a composition suitable for corneal application comprising at least one compound chosen from small leucine-rich repeat proteoglycans (SLRPs) in a physiologically compatible solution. The SLRPs are chosen from keratocan and mimecan, wherein the solution is buffered to a pH of from 6.5 to 8.5, and further wherein the concentration of the SLRP ranges from 10 to 500 µg/ml.

A sixth aspect of the present invention is directed to a composition suitable for corneal application comprising decorin in a physiologically compatible buffer solution, wherein the solution is buffered to a pH of from 6.8 to 8.5 and further wherein the concentration of the decorin ranges from 10 to 500 µg/ml.

A seventh aspect of the present invention is directed to a method of making a chemical composition suitable for corneal application, comprising the steps of dissolving fibril associated collagens with interrupted triple helices (FACITs) in a physiologically compatible solution to a concentration of FACITs that ranges from 10 to 500 µg/ml; and buffering the solution to a physiologically compatible pH level of from 6.5 to 8.5.

It is to be understood that both the foregoing general description and the following detailed description are exemplary and explanatory only and are not restrictive of the invention as claimed. Additional advantages will be set forth in the description which follows, and in part will be understood from the description, or may be learned by practice of the invention. The advantages and purposes of the invention may be obtained by means of the combinations set forth in the attached claims.

### DESCRIPTION OF THE PREFERRED EMBODIMENTS

Reference will now be made in detail to several embodiments of the invention. This application discloses compositions and teaches methods for making such compositions so that, when applied during or after the period of wearing the orthokeratology lens, the corneal corrective results are substantially maintained, thereby dispensing the need to wear rigid retainer contact lenses and the use thereof for the manufacture of a medicament for a therapeutic application to stabilize a reshaped unsoltened corneal tissue.

An accelerated orthokeratology procedure according to the present invention comprises the steps of wearing a pair of orthokeratology lenses to reshape the corneal tissues, and stabilizing the reshaped corneal tissues to retard relaxation of the cornea tissues to an original corneal curvature. The stabilizing agent renders permanent the reshaping of the corneal tissues. The accelerated orthokeratology procedure results in an emmetropic vision over a predetermined period of time, e.g., approximately 24 hours.

In the accelerated orthokeratology procedure, the patient, according to the present invention, may choose to wear the orthokeratology lenses, possibly in the evenings as retainer lenses, without applying the stabilizing agent to enjoy seeing through the reshaped corneal tissue. If the result is unsatisfactory, the patient may consult with an ophthalmologist or optometrist to examine the cornea and have a new pair of orthokeratology lenses fitted. When the patient is satisfied and decides to make this result permanent, the patient may apply the stabilizing agent to permanently stabilize the reshaped corneal tissues by retarding the relaxation of the corneal tissues to their original corneal curvature. The stabilizing agent rapidly restabilizes the corneal tissues in their new configuration after reshaping.

The orthokeratology lenses are preferably designed to gradually reshape the corneal tissue utilizing a mechanical pressure of the eyelid and a hydraulic pumping action of liquid tears, either natural or artificial.

According to the present application, at least two alternative types of compositions are disclosed which are suitable for use as a stabilizing agent. Each composition works independently and presents a different approach for stabilizing the corneal curvature. These compositions are generally characterized as a composition containing at least one FACIT or SLRP, or a transglutaminase enzyme composition.

According to a first approach of the present invention, a composition for the stabilizing agent is directed to a composition, preferably biologically compatible, suitable for stabilizing the collagen matrix of the eye. Corneal collagen fibrils are associated with natural binding macromoleclues. The stabilizing agent according to the present invention is generally composed of at least one compound selected from two such groups of macromolecules, namely fibril-associated collagens with interrupted triple helices, commonly abbreviated as "FACITs", and small leucine-rich repeat proteoglycans, abbreviated as "SLRPs."

In application, the extracellular matrix components of FACITs and SLRPs act as a stabilizing agent by cross-linking with corneal collagen fibrils and controlling the fibril diameter. The stabilizing agent may also form bridges and be involved in binding fibrils together to limit the ability of fibrils to glide past one another. These bridges allow some flexibility but limit the distance that fibrils can move.

Several types of collagens useful to form a biologically compatible collagenous reaction product of this invention can be chosen from a group including type VI collagen, type XX collagen, type XII collagen, and type XIV collagen. These types of collagens are members of the FACITs. The various types of FACITs can be extracted and purified or procured from academic sources, such as Schepens Eye Research Institute (Boston, MA).

The components of the stabilizing composition also include proteoglycans, such as SLRPs. Adding these SLRPs to the composition has been found to be effective in stabilizing the cornea. Despite its misleading nomenclature, SLRPs are large molecules. For example, several types of SLRPs can be chosen from decorin, keratocan, biglycan, epiphycan, lumican, mimican, and fibromodulin. Decorin includes dermatan sulfate glycosaminoglycan chains, and keratocan includes keratan sulfate glycosaminoglycan chains. Many SLRPs (such as biglycan, keratan, sulfate proteoglycan, deorin, laminin, and heparan sulfate proteoglycan) are also available from commercial sources, such as Sigma Chemical Company (Milwaukee, WI) ("Sigma"). Others are available from academic institutions or can be prepared by processes known in the art.

The following provides detailed descriptions of the various types of collagens in the FACIT family. Collagen proteins comprise a large, heterogeneous class of molecules that are associated with many developmental and physiological functions. At least twenty collagens have now been identified and about eleven of these are involved in the development of corneal structures. Two characteristics that all collagen molecules appear to have in common are being composed of three chains (termed α-chains) and having at least one domain in which these chains are arranged in a triple-helix. By convention, each collagen type is assigned a number (in Roman numerals), reflecting the chronological order in which it was discovered. FACITs play a major role in organizing corneal fibrillar collagens as discussed below.

It is known in the art that type VI collagen has a form of beaded filaments found in most stromal connective tissues. In the adult human corneal stroma, the molecule is present in quantities approaching that of the fibrillar collagens. These beaded filaments intertwine among, and appear to interact with the striated fibrils and corneal proteoglycans.

Studies have been done on the expression of the FACIT molecules types XII and XIV in the developing avian cornea. These studies, as detailed in "A New Fibril Associated Collagen, Type XX, Expressed in the Developing Avian Cornea," by Gordon et al. (Abstract in `Through the Looking Glass" Macromolecular Morphogenesis Symposium Sponsored by The Schepens Eye Research Institute, Boston, MA, January 22-23, 1999, hereinafter "Through the Looking Glass"), have identified new FACITs, including type XX, that may be involved in organizing corneal fibrils. These studies conclude that the developmental expression pattern of types XII, XIV, and XX collagen is correlated with the potential functions of these molecules in the avian cornea. The smaller amino terminal NC3 domains of type XX suggest that it may allow collagen fibrils closer proximity to each other.

It has been demonstrated in "Assembly of the Corneal Extracellular Matrix is a Multistep Process with Each Step Independently Regulated by Different Macromolecular Interactions," by David E. Birk (Abstract in "Through the Looking Glass"), that the proper development and maintenance of stromal architecture is required for corneal transparency. Fibril structure is one important determinant of corneal function. Collagen fibrils are initially assembled as intermediates, termed segments. These intermediates are incorporated into the developing extracellular matrix and as development proceeds a regulated maturation occurs. This fibril maturation involves the transformation of intermediates within the immature corneal stroma into longer, mature fibrils. In the cornea, the increase in length occurs with no change in diameter while in other tissues there is an increase in both length and diameter. The avian cornea has been used to study the regulation of collagen fibril formation. In the cornea, heterotypic interactions of collagen types I and V regulate the initial assembly of the fibril segment. Using a dominant-negative approach, the type V collagen composition determines the diameter of newly formed fibril segments. This effect is mediated by the amino terminal domain. Alterations in molecular assembly due to the presence of type V collagen limit fibril growth and favor the initiation of new fibril segments. This serves to increase the number of fibrils present during the rapid growth of the stroma.

The next step in fibril growth is an increase in length to yield the mature fibril. One study implicates the fibril-associated proteoglycans in the regulation of this step. One theory is that segments undergo a post-depositional fusion followed by molecular rearrangements that give rise to longer fibrils of the mature tissue. Based on this theory, an experiment was designed and predicted that, at different stages in development, there are temporal and spatial changes in components associated with the segment surface. These changes would serve to stabilize or destabilize the different steps in fibrillogenesis. Removing proteoglycans from the surface of isolated fibrils resulted in abnormal fibril growth, yielding longer and larger diameter fibrils. Adding back proteoglycans prevents this abnormal growth in a dose dependent manner.

In another study on the relation between "Type XII Collagen and Corneal Morphogenesis" by SundarRaj et al. (Abstract in "Through the Looking Glass"), it was determined that the developmentally regulated distribution of two alternatively spliced variants of type XII collagen might be associated with specific morphogenetic events involving tissue condensation.

The immunohistochemical analyses done by others indicated that the short valiant of type XII collagen was expressed in the rabbit corneal stroma and sclera from an early stage (day 14) of corneal development. The short form was also present in the connective tissue of the eyelids from an early stage of the eyelid fold formation. However, the long form was first expressed in the posterior stroma at a later stage (by day 24). During the later stages of pre- and postnatal development, the distribution of the long variant form progressed from the posterior to anterior stroma (a pattern similar to that of stromal condensation during corneal development). The long form was transiently expressed in subepidermal tissues in restricted regions where the eyelids had come together and fused. The appearance of type XII collagen in the corneal epithelial membrane ("BM") zone was evident just prior to the opening of the eyelids.

The study concludes that the differences in the temporal and spatial distribution of the long and the short variants of type XII collagen suggest that these variants may have different functions in corneal development. The long variant form of type XII collagen may be involved in corneal stromal condensation. Its interactions and function(s) in the BM zone are likely to be different than those in the stroma.

Therefore, the aforementioned studies related to the various types of FACITs indicate that these unique collagens interact with stromal collagen fibrils contributing to the organization of the corneal stroma to regulate fibril growth, provide corneal transparency and stability.

As previously discussed, adding SLRPs to the collagen mixture has been found to effectively stabilize the cornea. The following will discuss the SLRPs in further detail. In "Gene-Targeted Lumican-Deficient Mice: Regulation of Collagen Fibril Assembly and Corneal Transparency by Lumican," by S. Chakravarti (Abstract in "Through the Looking Glass"), a study was designed where corneal proteoglycans were hypothesized to be key regulators of a highly organized collagen matrix needed for transparency of the cornea. This study elucidated the role of lumican, a major corneal keratan sulfate ("KS") proteoglycan, in regulating collagen architecture and acquisition of corneal transparency. The study concluded that lumican played a critical role in collagen fibril assembly and corneal transparency. Progression of opacity might be a direct consequence of the age-dependent increase in collagen anomalies observed in the older mutants. A reduction in total KS content in lumican-deficient mice was likely to affect specific hydration and other physiological properties of the cornea.

in "Some Speculation on the Evolutionary Origin of the Genes for Corneal Keratan Sufate Proteoglycan Core Proteins," by G. W. Conrad et al. (Abstract in "Through the Looking Glass"), the scientists suggested that neither decorin nor lumican core protein genes were expressed in a cornea-specific manner, although lumican was glycosylated uniquely in the cornea. Mimecan was expressed in a more restricted array of tissues, whereas keratocan expression was completely, or almost completely, depending on species, cornea-specific. Sufficient genomic sequence data are now available from some vertebrates allowing comparison of the genomic structure of the mimecan gene (8 exons) with that of the keratocan gene (3 exons). Based on their nucleotide sequences, dendrograms of the family of genes expressing core proteins bearing keratan sulfate chains suggest that mimecan is distinctly more divergent in its sequence than keratocan. The primordial genome from which these genes evolved may have been simple, with introns appearing later (mimecan thus appearing late in evolution), or it may have evolved in exactly the reverse order, with primitive introns being eliminated with time (mimecan thus appearing early in evolution). Resolution of this question may derive from analysis of genomes of other organisms that have corneas.

To examine the cornea during corneal injury, a study on the relationship between "Growth Factors and Proteoglycans in the Stroma," by V. Trinkaus-Randall, et al. (Abstract in "Through the Looking Glass"), indicated that TGFβ played a role in the process of wound repair. TGFβ altered the composition of the extracellular matrix in stromal fibroblasts at the level of both core protein and GAG. The increase in perlecan might enhance the responsiveness of βFGF and alter its binding to cell surface.

In an experiment on the "Role of Lumican in Wound Healing of Mouse Corneal Epithelium" by W. W.-Y. Kao (Abstract in "Through the Looking Glass"), it was concluded that lumican might regulate epithelial cell migration, thus contributing to cornea epithelial wound healing.

In a study relating to the "Control of Matrix Assembly and Cell Growth by Proteoglycan Signaling," by R. V. lozzo (Abstract in "Through the Looking Glass"), it was shown that decorin, a prototype member of an expanding family of small leucine-rich proteoglycans, was directly involved in the control of matrix assembly primarily because of its ability to bind fibrillar collagen and to delay fibrillogenesis. The study also observed that decorin might act as a direct modulator of cell growth. For example, decorin levels are markedly elevated during growth arrest and quiescence, and its expression is abrogated by viral transformation. The study demonstrated that there was a direct interaction between decorin protein core and the EGF receptor. Therefore, the heightened biosynthesis of decorin by stromal elements in either wound healing or cancer growth might represent a natural mechanism of growth control.

Therefore, the aforementioned studies regarding SLRPs indicate that these unique matrix components bind to stromal collagen fibrils to control matrix organization and stabilize matrix composition.

According to the present invention, after the desired shape of corneal tissue has been achieved through an orthokeratological procedure, the stabilizing agent is applied to the corneal tissues to maintain the desired shape. This stablilizing agent should be applied directly to the corneal tissues. Thereafter, the shaping lens may be, but need not be, placed back into the corneal tissues until the stabilizing agent has had sufficient time to perform its function. As discussed below, it has been found that stabilizing agents of the present invention can perform their function before the cornea regresses to its former shape. However, with certain stabilizing agents, placing the orthokeratological lens back on the cornea for a day after application of the stabilizing agent may be advantageous.

### FACITs and SLRPs

In a first embodiment of the present invention FACITs are dissolved or suspended in a physiologically compatible buffer solution. Generally, the concentration of the FACITs will be in the range from 10 to 500 µg/ml. Preferably, the concentration of the FACITs will be in the range from 20 to 250 µg/ml, and most preferably, from 40 to 100 µg/ml. Also described is the addition of SLRPs, so that when SLRPs are also included either alone or in combination with the FACITs, the SLRPs have a concentration in the range, for example, from 10 to 500 µg/ml, from 20 to 25 µg/ml, from 40 to 100 µg/ml, and including any range of integers subsumed within these ranges.

The FACITS are diluted or dissolved in a buffer, such as a neutral pH phosphate buffer, having a concentration, for example, from 0.005 to 0.5M at a pH ranging from 6.5 to 8.5, and preferably from 6.8 to 7.6. Other suitable buffers include HEPES, TRIZMA® (Sigma-Aldrich, or other supplier of TRIS buffer), or other biologically suitable buffers known in the art, which may also be in a concentration from 0.005 to 5M.

This stabilizing agent solution is then applied to the cornea in the form of drops for a period of time from 1 day to 14 days during the period of or after wearing the orthokeratology lens. The buffer system and pH may be modified to optimize interaction with corneal collagens. For example, the buffer pH may be increased to 7.6 or higher in order to open up the collagen matrix structure to increase delivery of the composition to the eye tissue. As one of skill in the art would recognize, the pH is limited by the potential for damage to the eye tissue. In general, a suitable pH would not be above 8.0 because of the danger of such damage.

This solution interacts with collagen fibrils in the cornea to control fibril diameter by forming bridges to attach one collagen fibril to another adjacent fibril. These bridges allow fibril flexibility but restrain the distance that fibrils move. Thereafter, the corneal tissues will retain their shape without further use of reshaping lenses.

### Example 1:

A series of *ex vivo* laboratory experiments were performed on enucleated porcine cornea to optimize the effects of stabilization. Enucleated porcine eyes were preserved in Optisol Solution and stored at 2-8°C. Treated eyes were examined visually and using biomicroscopy to monitor the effects on corneal epithelium, stroma, corneal clouding, corneal rigidity, anterior chamber response, and corneal thickness. Treated eyes underwent mechanical testing to evaluate changes in low and high modulus to evaluate the stress and strain under compressive force, as described in detail below. A decrease in low modulus indicated softening or a reduction in force needed to compress the collagenous network due to increased ability to force out fluids. In contrast, an increase in low modulus indicated stiffening. Any changes in the high modulus indicated damage to the collagen fibril network.

In the first phase of these *ex vivo* experiments, a total of 12 porcine eyes are prescreened. Enucleated eyes are exposed to drops of the stabilizing agent comprised of rabbit cornea type VI collagen (available from the Schlepens Eye Institute) having a concentration of 1 mg/ml and buffered with 0.005M sodium phosphate to a pH of 7.2. The drops are applied over a 7-day period with the drops being applied twice each day after removal form Optisol and rinsing with balanced salt solution ("BSS") or phosphate buffered saline ("PBS"). Eyes are then returned to Optisol. All eyes are stored at room temperature and are exposed to natural light. Two concentrations of each agent are used for treatment. A slide is then applied to the corneal surface of the treated eyes and held in place using thumb pressure, or mechanically, generating a flattened surface. After 7 days, topographical analysis shows minimal rebound.

In the second phase of these *ex vivo* experiments, the same stabilizing agents are further evaluated in enucleated porcine eyes fitted with the orthokeratology lenses. The stabilizing agent is applied while wearing the orthokeratology lenses. Eyes are examined visually, by biomicroscopy, and using equipment to measure corneal curvature, such as keratometry and/or topography. The results of these experiments show minimal rebound after 7 days.

### Example 2:

The test eyes received both a molding treatment with a shaping lens followed by administration of decorin, an SLRP, having a concentration of 500 µg/ml buffered with 0.04M sodium phosphate to a pH of about 7.2. The solution was applied according to the following schedule: The mold was applied on Friday afternoon, and remained there until Monday morning when the molds were removed. After the mold removal, the eyes were then exposed to 100 µl of the decorin solution. The mold was reapplied onto each eye. Then each eye was placed back into Optisol solution. The application of decorin was repeated on Tuesday and on Wednesday.

The mold was reapplied to one eye. Subjective photographic assessment of that eye showed that the treatment with decorin with the mold reapplied resulted in a flat cornea with no rebound.

With respect to the second eye, after the eye was exposed to the decorin on Wednesday, the mold was not reapplied. This was done to determine if the eye would rebound back to its original shape. After three more days, subjective photographic assessment of that eye showed that there was negligible to no rebound of the corneal tissues.

### Example 3:

The test eyes received both molding treatment with shaping lens followed by administration of keratan sulfate, an SLRP, having a concentration of 100 µg/ml buffered with 0.04M disodium phosphate to a pH of about 7.2. The solution was applied according to the following schedule: The mold was applied on Friday afternoon, and remained there until Monday morning when the molds were removed. After the mold removal, the eyes were then exposed to 100µl. The mold was reapplied onto each eye. Then each eye was placed back into Optisol solution. The application of keratan sulfate was repeated on Tuesday and on Wednesday.

The mold was reapplied to one eye. Subjective photographic assessment of that eye showed that the treatment with keratan sulfate with the mold reapplied resulted in a flat cornea with no rebound.

With respect to the second eye, after the eye was exposed to the keratan sulfate on Wednesday, the mold was not reapplied. This was done to determine if the eye would rebound back to its original shape. After three more days, subjective photographic assessment of that eye showed that there was moderate rebound of the corneal tissue.

### Transglutaminase

According to a second approach to stabilizing the cornea of the present invention, the stabilizing agent, also preferably biologically compatible, may be derived from an enzyme normally found in the tissue, such as epidermis. The enzyme may include transglutaminase, for example, transglutaminase (Factor XIII) crosslinks proteins which are formed by covalent bonds between lysine and glutamine residues.

Transglutaminase mixed with EDTA in a range from, for example, 0.01 M to 0.25M or from 0.05M to 0.15M. The concentration of the transglutaminase may range from 0.01 M to 0.25M or from 0.05M to 0.1 M, and including any range of integers subsumed within these ranges. As transglutaminase requires calcium ions to become activated, EDTA is added to the stabilizing agent solution in order to bind calcium ions that might induce premature transglutaminase activation.

The solution should be buffered to a pH range from, for example, 6.8 to 7.6, or from 7.0 to 7.2, or any range of integers subsumed within these ranges. Suitable buffers include glycine buffer, TRIS buffer, and Bicine buffers. However, the buffer should not contain phosphates as phosphates have a tendency to precipitate in the presence of calcium. Then, calcium chloride in a range from 5 mM to 50 mM, preferably from 20 mM to 30 mM, is added to the transglutaminase solution as a catalyst. The delivery system will comprise a two-part device to add the components during application to the cornea. Such a device will enable the user to combine the calcium catalyst with the transglutaminase solution at the appropriate time without the need for separate solutions or two separate devices.

### Example 4:

The transglutaminase composition was prepared by adding 10mg of transglutaminase to 10mL of sterile water containing 2% sucrose, 0.1 M EDTA, and 5mM glycine buffer, pH 7.2. The transglutaminase was obtained from Sigma, Catalog No. T-5398, with a guinea pig liver or human recombinant source. The components were mixed and then diluted to 90 ml with sterile water. A second solution contained 25mM calcium chloride.

Prior to application to the cornea, 10mL of the calcium chloride solution was added to the transglutaminase composition and gently mixed. Droplets of this mixture were added to the surface of the cornea, just like adding eye drops, and allowed to react for about 10 minutes. The eye was then washed with sterile water or sterile buffer, e.g., "BSS," to remove excess transglutaminase mixture. As a result, the transglutaminase mixture mediated covalent crosslinks between various primary amine groups of peptide-bound lysines of collagen in the corneal stroma.

The above composition also went through a series of ex *vivo* laboratory experiments on enucleated porcine cornea to optimize the effects of stabilization. Enucleated porcine eyes were placed in ice until treated. Prior to treatment, each eye was placed in a bracket for stability and subjected to topographical evaluation using the Optikon 2000 system. Six topographs of each eye were taken and true composites generated. The corneal surface was dried using sterile gauze and then wetted with drops of 0.02M disodium phosphate. The wetted eyes were again dried and exposed to drops of 0.02M disodium phosphate. A glass slide was balanced on the surface of the cornea. Solutions of transglutaminase and calcium chloride (CaCl₂) were prepared in 50mM TRIS buffer, pH 8.5. The pH of TRIS buffer was adjusted to 8.5 by adding 2.5N sodium hydroxide (NaOH). Transglutaminase was prepared at 1 mg/mL in 10mL of TRIS buffer. CaCl₂ was prepared at a concentration of 25mM in 50mL of TRIS buffer.

Prior to administration, 1 mL of CaCl₂ solution was mixed with 9mL of transglutaminase solution because transglutaminase requires Ca⁺⁺ as a catalyst. The transglutaminase/CaCl₂ solution was added dropwise to the area around the glass slide. Approximately 1 mL of enzyme solution was applied in a period of 2 minutes. The slide was then removed and the eye washed with 0.004M phosphate buffer, at pH 7.4. The eye was then reexamined topographically and photos taken. Following the topographical evaluation, the eyes were placed in Optisol for storage pending additional evaluations. Three eyes were treated using this protocol.

There were some difficulties in treating the first two eyes due to the difficulty in applying the enzyme solution while balancing the glass slide. In the third attempt, drops of transglutaminase were applied to the cornea and the slide applied to the corneal surface and held in place using thumb pressure. Drops of enzyme solution were subsequently applied to corneal surfaces around the glass slide. No flattening effect was noted in the first two eyes. The topographical results from the first eye appeared to show corneal steepening as shown in Table 1 below. However, topographical maps clearly demonstrated a flattening of the central cornea in the third eye. Refractive power was reduced by approximately 1.5 diopters. All eyes appeared clear by visual examination. Eyes were placed in Optisol for storage.

**Table 1: Corneal Power as Measured by Topographical Mapping**

| Porcine Eye No. | Pretreatment (in Diopter) | | Post-treatment (in Diopter) | |
|---|---|---|---|---|
| 1 | 38.98 | 37.33 | 42.14 | 39.2 |
| 2 | 39.89 | 37.7 | 39.98 | 37.26 |
| 3 | 40.25 | 38.16 | 38.79 | 36.83 |

After treatment, corneal buttons were dissected, placed in Optisol and shipped to Rutgers University for stress-strain analysis. In the stress-strain analysis, corneal buttons were placed on a slightly convex surface and exposed to compressive forces. Stress-strain curves represent the force per unit area of cross-section required to compress the cornea a certain amount as expressed in percentage. Resultant curves indicate several distinct phases. The lower part (low modulus region) represents the resistance to squeeze out fluid between collagen fibrils. The middle part, wherein the stress-strain curve does not change, and the upper part (high modulus region) represent compression of collagen fibrils. A reduction in low modulus indicates that the cornea is softer. An increase indicates that the corneal buttons are stiffer and have been stabilized.

Transglutaminase treatment gave encouraging results. Topographical evaluation indicated that one porcine eye treated with transglutaminase following corneal flattening using a glass slide exhibited a refractive power reduction of about 1.5 diopters after removal of the glass slide. It is important to note that two additional eyes were included in this treatment series. Glass slides were also applied to these porcine eyes. However, enzyme addition was applied with the eyes in the horizontal position and did not appear to flow under the glass slide into the cornea. In these eyes, there was no evidence of a reduction in refractive power by topographical evaluation. Since these eyes did not show flattening of the central cornea following the application of the glass slide, it was unlikely that the corneal flattening observed in the successful eye was solely a result of the application of the glass slide.

### Example 5:

Additional experiments are performed to demonstrate that transglutaminase drops could effectively stabilize the cornea during orthokeratology. At least three different levels of transglutaminase are evaluated, including 10 mg/mL, 5 mg/mL, and 1 mg/mL. Three eyes per treatment are included, along with 2 sham controls, i.e., lens application with buffer drops only without transglutaminase, and one control with no treatment at all.

In this series, the experimental procedure includes placing the eyes in bracket holders, and conducting a topographical evaluation, 6 readings for each eye. Then, flattening lenses are applied and gently held in place using rubber band or other similar device. The eyes are positioned approximately 45° from horizontal, and conditioned with drops of 0.05M TRIS HCl solution, pH 7.6. While allowing the buffer to interact for a minute, transglutaminase solution is mixed with the CaCl₂ solution in a 9 to 1 ratio. Thereafter, the eyes are treated with calcium containing transglutaminase drops, each drop containing approximately 0.1 mL of solution, at intervals of 30 seconds for two minutes, followed by applying drops of 0.05M TRIS HCl buffer. The eyes are then topographically evaluated, examined using slit-lamp biomicroscopy, dissected from the globes, and are then shipped to Rutgers University for compression analysis.

Topographic evaluation shows a decrease of 1.5 to 2.0 in diopter power, and an increase in low modulus, showing a stiffening of the corneal tissue.

### Example 6:

The test eyes received both molding treatment with shaping lens followed by administration of transglutaminase solution having a concentration of 1 mg/ml transglutaminase buffered with 0.05M TRIS HCl to a pH of about 7.2, plus calcium chloride in 0.05M TRIS HCl buffer at a concentration of 25mM. The solution was applied according to the following schedule: The mold was applied on Friday afternoon, and remained there until Monday morning when the molds were removed. After the mold removal, the eyes were then exposed to 100µl of the transglutaminase solution. The mold was reapplied onto each eye. Then each eye was placed back into Optisol solution. The application of transglutaminase solution was repeated on Tuesday and on Wednesday.

The mold was reapplied to one eye. Subjective photographic assessment of that eye showed that the treatment with transglutaminase with the mold reapplied resulted in a flat cornea with no rebound.

With respect to the second eye, after the eye was exposed to the transglutaminase on Wednesday, the mold was not reapplied. This was done to determine if the eye would rebound back to its original shape. After three more days, subjective photographic assessment of that eye showed that there was only slight rebound of the corneal tissues.

In summary, the present invention teaches various compositions having an eye-drop delivery system, for use during or after wearing orthokeratology lens to maintain or stabilize corneal tissue corrected by the accelerated orthokeratology procedure. The composition is applied to the cornea to bind collagen fibrils in the cornea, to control fibril diameter, and to form bridges between collagen fibrils.

The present invention anticipates that it can be used to stabilize the corneas of patients having already undergone orthokeratology. The stabilization procedure will eliminate the need to continue wearing retainer lenses to maintain the shape of the cornea. While it may be possible to simply utilize the stabilization step for these patients, it is anticipated that the cornea will have to be destabilized before it can be restabilized to take on the new configuration.

### Example 7:

### Protocol for Toxicity Evaluation of Decorin in the Reline Eye

The purpose of the following evaluation was to determine if (1) there is toxicity associated with the use of decorin on the eye; (2) assess the penetration of decorin into the cornea; and (3) quantitate decorin in the cornea following exogenous application of decorin.

One, three, and five daily applications of decorin were assessed using female cats (6 months to 2 years of age) with normal corneas as the model system. The decorin was obtained as a dry powder (Sigma) and reconstituted in a 0.1 M phosphate buffer. In order to perform the microscopic evaluations, decorin was labeled with Oregon Green 514 using a commercially available kit from Molecular Probes.

Five cats were used in the study. Each cat was sedated prior to topical application of medication or photography of the eye. All animals received an ocular examination and photographs (whole eye, slit lamp, and endothelial cells) prior to treatment. Eyes were randomly assigned to a treatment group. The decorin was applied to the interior of a contact lens, and the lens placed on the cat's eye. The lens remained on the eye for 10 minutes. All animals were observed daily during the study. Three eyes were randomly assigned to a treatment or control group. At least 2 more eyes were obtained for use as controls for each of the histograph, TEM, and confocal microscopy evaluations.

One eye from each of the three treatment groups was treated with Oregon Green 5149 labeled decorin.
◆ Treatment group 1 eyes received one application of 50ug of decorin in 100ul buffer on day 1. Photographs and exams were obtained just after treatment and again on days 2, 4, and 8-post treatment. Exams and photos were then done weekly for the remainder of the month.
◆ Treatment group 2 eyes received one application of 50ug decorin in 100ul buffer on days 1, 2, and 3. Photographs and exams were obtained just after treatment and again on days 2, 3, 5, and 8. Thereafter, exams and photos were obtained weekly for the remainder of the month.
◆ Treatment group 3 eyes received one application of 50ug decorin in 100ul of buffer on days 1, 2, 3, 4, and 5. Photographs and exams were obtained daily and again on day 8. Thereafter, exams and photos were obtained weekly for the remainder of the month.
◆ All animals were euthanized at one month. The eyes were enucleated. Each eye was cut in half. One half was fixed in formalin for histolgical analysis (H&E stain) of toxicity. The second half was further divided in half, one section was used for TEM visualization of the decorin, and the remainder was examined using confocal microscopy for those cats treated with labeled decorin.

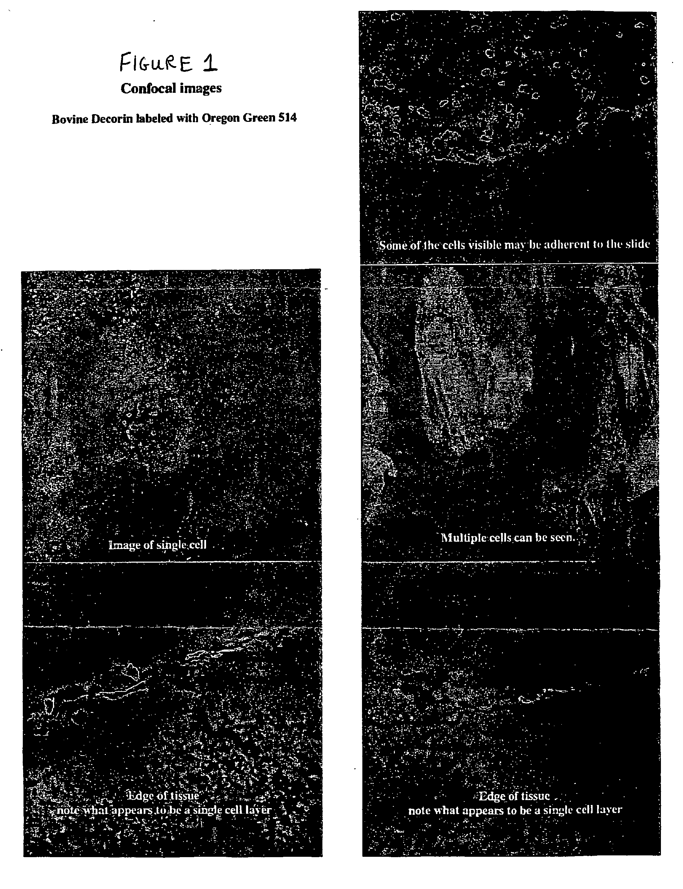

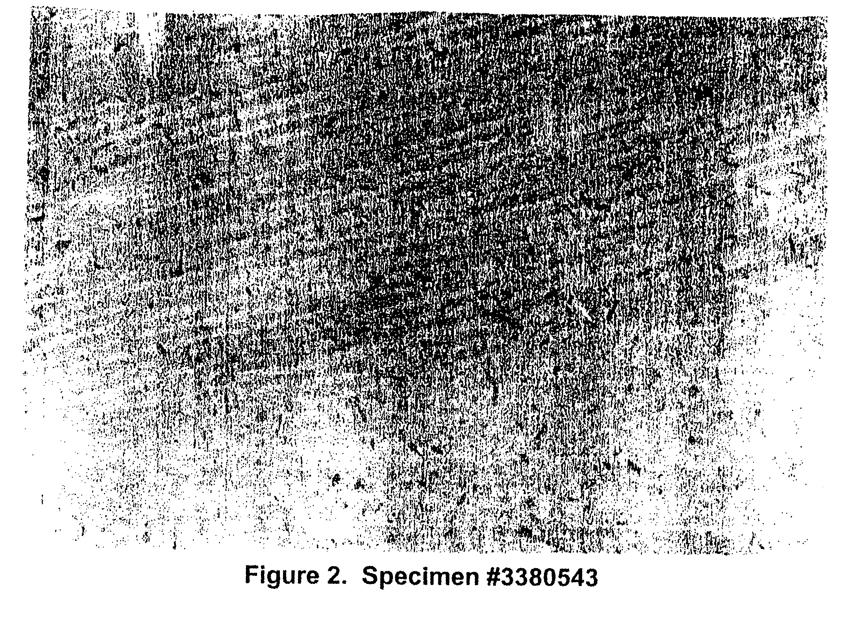

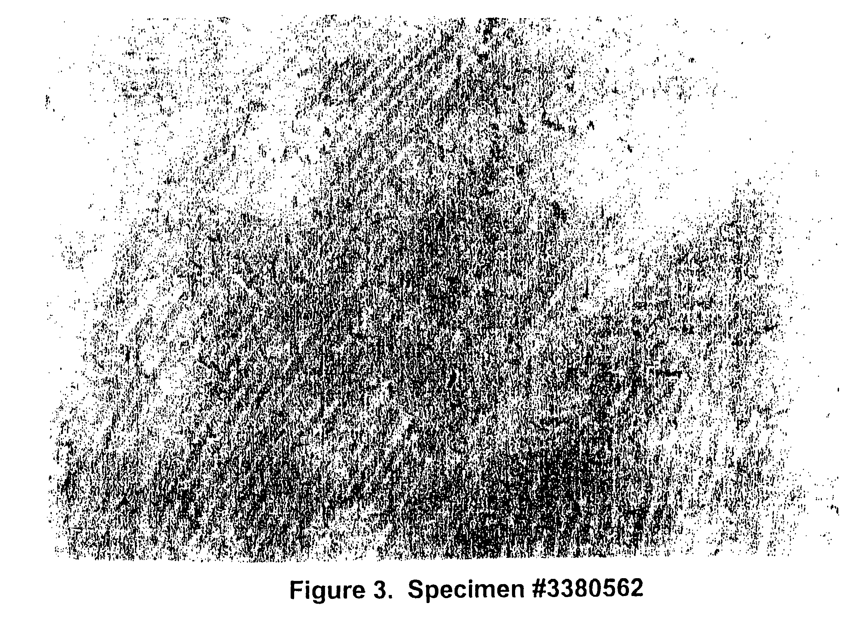

The results depicted in Figure 1 (confocal micrographs) illustrate that the decorin is penetrating the corneal tissue of the eye. In addition, Figure 2 (specimen # 33080543) shows that a cornea treated five times with decorin according to the treatment protocol above (treatment group 3) contained more collagen fibril-associated decorin than the untreated cornea shown in Figure 3 (specimen #3380562). Initial qualitative analysis indicates that the decorin filaments in the treated eye appear longer and "fatter". These "fatter" filaments were observed throughout the stormal sections, including the epithelial region, mid-stroma, and endothelial regions.

These observations indicate that exogenously added decorin can penetrate the corneal tissue of the eye and it can bind to the stromal matrix. In addition, there does not appear to be any ocular toxicity or adverse events associated with exogenous decorin application to the feline cornea.

It will be apparent to those skilled in the art that various modifications and variations can be made in the composition, the method for making the composition and method for applying such composition in the present invention, as well as other aspects of the invention without departing from the scope of the invention.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims and their equivalents.

## Claims

1. Use of a stabilizing agent for the manufacture of a medicament for a therapeutic application to stabilize a reshaped unsoftened corneal tissue which is corrected in an orthokeratological procedure to correct a patient's corneal curvature, wherein the stabilizing agent comprises at least one compound chosen from small leucine-rich repeat proteoglycans ("SLRP"), and fibril associated collagens with interrupted triple-helixes ("FACIT").

2. Use of the stabilizing agent as defined in claim 1 wherein the stabilizing agent is adapted to be applied to the corneal tissues of a patient.

3. Use of the stabilizing agent as defined in claim 1, wherein the stabilizing agent is adapted to interact with collagen fibrils to form bridges connecting the collagen fibrils.

4. Use of the stabilizing agent as defined in claim 3, wherein the stabilizing agent is adapted to control a fibril diameter of the cornea.

5. Use of the stabilizing agent as defined in claim 3, wherein the stabilizing agent is adapted that the bridges are sufficiently flexible to allow fibril movement within a predetermined range.

6. Use of the stabilizing agent as defined in claim 1, wherein the FACITs are chosen from type VI, type XII, type XIV, type XX, and any combination thereof.

7. Use of the stabilizing agent as defined in claim 1, wherein the SLRPs are chosen from biglycan, decorin, epiphycan, keratocan, lumican, mimecan, fibromodulin, and any combination thereof.

8. Use of the stabilizing agent as defined in claim 1, wherein the concentration of the stabilizing agent is within a range from 40 ug/ml to 100 µg/ml.

9. Use of the stabilizing agent as defined in claim 1, wherein the medicament is in a form of an eye-drop solution.

10. Use of the stabilizing agent as defined in claim 1, wherein the stabilizing agent is adapted to be applied after removing of a orthokeratological lens.

11. Use of the stabilizing agent as defined in claim 1, wherein the stabilizing agent is adapted to be applied before removing of a orthokeratological lens.

12. A method for making a chemical composition for application on a reshaped cornea resulting from an orthokeratology procedure, comprising the steps of:
a. preparing transglutaminase in a buffer solution;
b. mixing the solution and diluting in sterile water;
c. adding EDTA to the solution; and then
d. adding calcium chloride prior to application of the composition to the cornea.

13. An orthokeratological system, comprising:
a) an orthokeratological lens configured to be inserted into the patient's eye to reshape unsoftened corneal tissue into a preselected corneal shape dictated by the orthokeratological lens; and
b) a stabilizing agent to be administered into the patient's eye to stabilize the corneal tissue in the preselected shape wherein the stabilizing agent comprises at least one compound chosen from transglutaminase, SLRPs, and FACITs.

14. A composition suitable for corneal application comprising at least one compound chosen from fibril associated collagens with interrupted triple helices (FACITs) in a physiologically compatible solution, wherein the solution is buffered to a pH of from 6.5 to 8.5, and further wherein the concentration of the FACIT ranges from 10 to 500 µg/ml.

15. The composition of claim 14, wherein the FACITs are chosen from type VI, type XII, type XIV, type XX, and any combination, thereof,

16. The composition of claim 14, wherein the solution is buffered to a pH of from 6.8 to 7.6.

17. The composition of claim 14, wherein the concentration of the FACITs ranges from 40 to 100 µg/ml.

18. The composition of claim 14, wherein the composition further comprises at least one compound chosen from small leucine-rich repeat proteoglycans (SLRPs).

19. A composition suitable for corneal application comprising at least one compound chosen from small leucine-rich repeat proteoglycans (SLRPs) in a physiologically compatible solution, wherein the SLRPs are chosen from keratocan and mimecan, wherein the solution is buffered to a pH of from 6.5. to 8.5, and further wherein the concentration of the SLRP ranges from 10 to 500 µg/ml.

20. The composition of claim 19, wherein the solution is buffered to a pH of from 6.8 to 7.6.

21. The composition of claim 19, wherein the concentration of the SLRPs ranges from 40 to 100 µg/ml,

22. A composition suitable for corneal application comprising decorin in a physiologically compatible buffer solution, wherein the solution is buffered to a pH of from 6.8 to 8.5 and further wherein the concentration of the decorin ranges from 10 to 500 µg/ml.

23. The composition of claim 22, wherein the solution is buffered to a pH of from 6.8 to 7.6,

24. The composition of claim 22, wherein the concentration of the SLRPs ranges from 40 to 100 µg/ml.

25. The composition of claim 22, wherein the solution comprises a buffer chosen from phosphate buffers, HEPES and TRIZMA buffers.

26. The composition of claim 22, wherein the buffer concentration is from 0.005 to 5M.

27. A method of making a chemical composition suitable for corneal application, comprising the steps of:
a. dissolving fibril associated collagens with interrupted triple helices (FACITs) in a physiologically compatible solution to a concentration of FACITs that ranges from 10 to 500 µg/ml; and
b. buffering the solution to a physiologically compatible pH level of from 6.5 to 8.5.

28. The method of claim 27, wherein the concentration of the chemical composition is within a range from 40 µg/ml to 100 µg/ml for FACITs.

## Patentansprüche

1. Verwendung eines Stabilisierungsmittels für die Herstellung eines Medikaments für eine therapeutische Anwendung, um ein neu geformtes nicht erweichtes Hornhautgewebe, welches in einem orthokeratologischen Verfahren korrigiert wird, um die Hornhautkrümmung eines Patienten zu korrigieren, zu stabilisieren, wobei das Stabilisierungsmittel mindestens eine Verbindung umfasst, die aus kleinen Leucin-reichen Repeat-Proteoglykanen ("SLRP") und Fibrillen-assoziierten Kollagenen mit unterbrochenen Triple-Helices ("FACIT") ausgewählt wird.

2. Verwendung des Stabilisierungsmittels wie in Anspruch 1 definiert, wobei das Stabilisierungsmittel angepasst ist, um auf die Hornhautgewebe eines Patienten aufgetragen zu werden.

3. Verwendung des Stabilisierungsmittels wie in Anspruch 1 definiert, wobei das Stabilisierungsmittel angepasst ist, um mit Kollagenfibrillen zu wechselwirken, um Brücken zu bilden, die die Kollagenfibrillen verbinden.

4. Verwendung des Stabilisierungsmittels wie in Anspruch 3 definiert, wobei das Stabilisierungsmittel angepasst ist, um ein Fibrillen-Durchmesser der Hornhaut zu kontrollieren.

5. Verwendung des Stabilisierungsmittels wie in Anspruch 3 definiert, wobei das Stabilisierungsmittel angepasst ist, so dass die Brücken ausreichend flexibel sind, um Fibrillen-Bewegung innerhalb eines vorbestimmten Bereichs zu ermöglichen.

6. Verwendung des Stabilisierungsmittels wie in Anspruch 1 definiert, wobei die FACITs aus Typ VI, Typ XII, Typ XIV, Typ XX und irgendeiner Kombination davon ausgewählt werden.

7. Verwendung des Stabilisierungsmittels wie in Anspruch 1 definiert, wobei die SLRPs aus Biglycan, Decorin, Epiphycan, Keratocan, Lumican, Mimecan, Fibromodulin und irgendeiner Kombination davon ausgewählt werden.

8. Verwendung des Stabilisierungsmittels wie in Anspruch 1 definiert, wobei die Konzentration des Stabilisierungsmittels innerhalb eines Bereichs von 40 µg/ml bis 100 µg/ml ist.

9. Verwendung des Stabilisierungsmittels wie in Anspruch 1 definiert, wobei das Medikament in einer Form einer Augentropfenlösung ist.

10. Verwendung des Stabilisierungsmittels wie in Anspruch 1 definiert, wobei das Stabilisierungsmittel angepasst ist, um nach der Entfernung einer orthokeratologischen Linse angewendet zu werden.

11. Verwendung des Stabilisierungsmittels wie in Anspruch 1 definiert, wobei das Stabilisierungsmittel angepasst ist, um vor der Entfernung einer orthokeratologischen Linse angewendet zu werden.

12. Ein Verfahren zur Herstellung einer chemischen Zusammensetzung für die Anwendung auf einer neu geformten Hornhaut, die aus einem orthokeratologischen Verfahren resultiert, das die Schritte umfasst:
a. Herstellen von Transglutaminase in einer PufferLösung;
b. Mischen der Lösung und Verdünnen im sterilem Wasser;
c. Hinzugeben von EDTA zu der Lösung; und dann
d. Hinzugeben von Calciumchlorid vor der Anwendung der Zusammensetzung auf der Hornhaut.

13. Ein orthokeratologisches System, das umfasst:
a) eine orthokeratologische Linse, die konfiguriert ist, um in das Auge des Patienten eingesetzt zu werden, um nicht erweichtes Hornhautgewebe in eine zuvor ausgewählte Hornhautform, die durch die orthokeratologische Linse vorgeschrieben ist, neu zu formen; und
b) ein Stabilisierungsmittel, das in das Auge des Patienten verabreicht wird, um das Hornhautgewebe in der zuvor ausgewählten Form zu stabilisieren, wobei das Stabilisierungsmittel mindestens eine Verbindung umfasst, die aus Transglutaminase, SLRPs und FACITs ausgewählt wird.

14. Eine Zusammensetzung, die für Hornhaut-Anwendung geeignet ist, die mindestens eine Verbindung, die aus Fibrillen-assoziierten Kollagenen mit unterbrochenen Triple-Helices (FACITs) ausgewählt wird, in einer physiologisch kompatiblen Lösung umfasst, wobei die Lösung auf einen pH von 6.5 bis 8.5 gepuffert ist und wobei weiter die Konzentration des FACIT von 10 bis 500 µg/ml reicht.

15. Die Zusammensetzung nach Anspruch 14, wobei die FACITs aus Typ VI, Typ XII, Typ XIV, Typ XX und irgendeiner Kombination davon ausgewählt werden.

16. Die Zusammensetzung nach Anspruch 14, wobei die Lösung auf einen pH von 6.8 bis 7.6 gepuffert ist.

17. Die Zusammensetzung nach Anspruch 14, wobei die Konzentration der FACITs von 40 bis 100 µg/ml reicht.

18. Die Zusammensetzung nach Anspruch 14, wobei die Zusammensetzung weiter mindestens eine Verbindung umfasst, die aus kleinen Leucin-reichen Repeat-Proteoglykanen (SLRPs) ausgewählt wird.

19. Eine Zusammensetzung, die für Hornhaut-Anwendung geeignet ist, die mindestens eine Verbindung, die aus kleinen Leucin-reichen Repeat-Proteoglykanen (SLRPs) ausgewählt wird, in einer physiologisch kompatiblen Lösung umfasst, wobei die SLRPs aus Keratocan und Mimecan ausgewählt werden, wobei die Lösung auf einen pH von 6.5 bis 8.5 gepuffert ist und wobei weiter die Konzentration des SLRP von 10 bis 500 µg/ml reicht.

20. Die Zusammensetzung nach Anspruch 19, wobei die Lösung auf einen pH von 6.8 bis 7.6 gepuffert ist.

21. Die Zusammensetzung nach Anspruch 19, wobei die Konzentration der SLRPs von 40 µg/ml bis 100 µg/ml reicht.

22. Eine Zusammensetzung, die für Hornhaut-Anwendung geeignet ist, die Decorin in einer physiologisch kompatiblen Pufferlösung umfasst, wobei die Lösung auf einen pH von 6.8 bis 8.5 gepuffert ist und wobei weiter die Konzentration des Decorins von 10 bis 500 µg/ml reicht.

23. Die Zusammensetzung nach Anspruch 22, wobei die Lösung auf einen pH von 6.8 bis 7.6 gepuffert ist.

24. Die Zusammensetzung nach Anspruch 22, wobei die Konzentration der SLRPs von 40 bis 100 µg/ml reicht.

25. Die Zusammensetzung nach Anspruch 22, wobei die Lösung einen Puffer umfasst, der aus Phosphatpuffern, HEPES- und TRIZMA-Puffern ausgewählt wird.

26. Die Zusammensetzung nach Anspruch 22, wobei die Pufferkonzentration 0.005 bis 5M ist.

27. Ein Verfahren zur Herstellung einer chemischen Zusammensetzung, die für Hornhaut-Anwendung geeignet ist, das die Schritte umfasst:
a. Auflösen von Fibrillen-assoziierten Kollagenen mit unterbrochenen Triple-Helices (FACITs) in einer physiologisch kompatiblen Lösung bis zu einer Konzentration, die von 10 bis 500 µg/ml reicht; und
b. Puffern der Lösung auf ein physiologisch kompatibles pH Niveau von 6.5 bis 8.5.

28. Das Verfahren nach Anspruch 27, wobei die Konzentration der chemischen Zusammensetzung innerhalb eines Bereichs von 40 µg/ml bis 100 µg/ml für FACITs ist.

## Revendications

1. Utilisation d'un agent stabilisateur pour la fabrication d'un médicament destiné à l'application thérapeutique pour stabiliser un tissu de la cornée non ramolli remis en forme qui est corrigé dans une procédure orthokératologique pour corriger la courbure de la cornée d'un patient, où l'agent stabilisateur comprend au moins un composé choisi parmi les petits protéoglycanes répétés riches en leucine (« SLRP »), et les collagènes associés à des fibrilles avec triples hélices interrompues (« FACIT »).

2. Utilisation de l'agent stabilisateur selon la revendication 1, dans laquelle l'agent stabilisateur est conçu pour être appliqué aux tissus de la cornée d'un patient.

3. Utilisation de l'agent stabilisateur selon la revendication 1, dans laquelle l'agent stabilisateur est conçu pour interagir avec des fibrilles de collagène pour former des ponts raccordant les fibrilles de collagène.

4. Utilisation de l'agent stabilisateur selon la revendication 3, dans laquelle l'agent stabilisateur est conçu pour maîtriser un diamètre de fibrille de la cornée.

5. Utilisation de l'agent stabilisateur selon la revendication 3, dans laquelle l'agent stabilisateur est conçu pour que les ponts soient suffisamment flexibles pour permettre un mouvement de fibrille dans une gamme prédéterminée.

6. Utilisation de l'agent stabilisateur selon la revendication 1, dans laquelle les FACIT sont choisis parmi le type VI, le type XII, le type XIV, le type XX et l'une quelconque de leurs combinaisons.

7. Utilisation de l'agent stabilisateur selon la revendication 1, dans laquelle les SLRP sont choisis parmi le biglycane, la décorine, l'épiphycane, le kératocane, le lumicane, le mimécane, la fibromoduline et l'une quelconque de leurs combinaisons.

8. Utilisation de l'agent stabilisateur selon la revendication 1, dans laquelle la concentration de l'agent stabilisateur est dans une gamme de 40 µg/mL à 100 µg/mL.

9. Utilisation de l'agent stabilisateur selon la revendication 1, dans laquelle le médicament se présente sous la forme d'une solution de gouttes pour les yeux.

10. Utilisation de l'agent stabilisateur selon la revendication 1, dans laquelle l'agent stabilisateur est conçu pour être appliqué après enlèvement d'une lentille orthokératologique.

11. Utilisation de l'agent stabilisateur selon la revendication 1, dans laquelle l'agent stabilisateur est conçu pour être appliqué avant d'enlever une lentille orthokératologique.

12. Procédé de préparation d'une composition chimique pour application sur une cornée remise en forme résultant d'une procédure d'orthokératologie, comprenant les étapes consistant à :
a. préparer de la transglutaminase dans une solution tampon;
b. mélanger la solution et diluer dans de l'eau stérile;
c. ajouter de l'EDTA à la solution ; puis
d. ajouter du chlorure de calcium avant application de la composition à la cornée.

13. Système orthokératologique comprenant :
a) une lentille orthokératologique configurée pour être insérée dans l'oeil d'un patient pour remettre en forme le tissu de la cornée non ramolli sous une forme de cornée présélectionnée dictée par la lentille orthokératologique ; et
b) un agent stabilisateur à administrer dans l'oeil du patient pour stabiliser le tissu de la cornée sous la forme présélectionnée, où l'agent stabilisateur comprend au moins un composé choisi parmi la transglutaminase, les SLRP et les FACIT.

14. Composition appropriée pour une application à la cornée comprenant au moins un composé choisi parmi les collagènes associés à des fibrilles avec triples hélices interrompues (FACIT) dans une solution physiologiquement compatible, où la solution est tamponnée à un pH de 6,5 à 8,5, et où en outre, la concentration du FACIT varie de 10 à 500 µg/mL.

15. Composition selon la revendication 14, dans laquelle les FACIT sont choisis parmi le type VI, le type XII, le type XIV, le type XX et l'une quelconque de leurs combinaisons.

16. Composition selon la revendication 14, dans laquelle la solution est tamponnée à un pH de 6,8 à 7,6.

17. Composition selon la revendication 14, dans laquelle la concentration des FACIT varie de 40 à 100 µg/mL.

18. Composition selon la revendication 14, dans laquelle la composition comprend en outre au moins un composé choisi parmi les petits protéoglycanes répétés riches en leucine (SLRP).

19. Composition appropriée pour une application à la cornée comprenant au moins un composé choisi parmi les petits protéoglycanes répétés riches en leucine (SLRP) dans une solution physiologiquement compatible, où les SLRP sont choisis parmi le kératocane et le mimécane, où la solution est tamponnée à un pH de 6,5 à 8,5, et où en outre, la concentration du SRLP varie de 10 à 500 µg/mL.

20. Composition selon la revendication 19, dans laquelle la solution est tamponnée à un pH de 6,8 à 7,6.

21. Composition selon la revendication 19, dans laquelle la concentration des SLRP varie de 40 à 100 µg/mL.

22. Composition appropriée pour une application à la cornée comprenant de la décorine dans une solution tampon physiologiquement compatible, où la solution est tamponnée à un pH de 6,8 à 8,5, et où en outre, la concentration de la décorine varie de 10 à 500 µg/mL.

23. Composition selon la revendication 22, dans laquelle la solution est tamponnée à un pH de 6,8 à 7,6.

24. Composition selon la revendication 22, dans laquelle la concentration des SLRP varie de 40 à 100 µg/mL.

25. Composition selon la revendication 22, dans laquelle la solution comprend un tampon choisi parmi les tampons de phosphate, les tampons HEPES et TRIZMA.

26. Composition selon la revendication 22, dans laquelle la concentration du tampon est de 0,005 à 5 M.

27. Procédé de préparation d'une composition chimique appropriée pour une application à la cornée, comprenant les étapes consistant à :
a. dissoudre des collagènes associés à des fibrilles avec triples hélices interrompues (FACIT) dans une solution physiologiquement compatible à une concentration de FACIT qui varie de 10 à 500 µg/mL; et
b. tamponner la solution à un niveau de pH physiologiquement compatible de 6,5 à 8,5.

28. Procédé selon la revendication 27, dans lequel la concentration de la composition chimique est dans une gamme de 40 µg/mL à 100 µg/mL pour les FACIT.
